(19) **Europäisches Patentamt**
**European Patent Office**
**Office européen des brevets**

(11) **EP 1 992 270 B1**

(12) **EUROPEAN PATENT SPECIFICATION**

(45) Date of publication and mention
of the grant of the patent:
**14.03.2012 Bulletin 2012/11**

(51) Int Cl.:
*A61B 5/00* $^{(2006.01)}$       *A61B 1/00* $^{(2006.01)}$
*A61B 1/04* $^{(2006.01)}$

(21) Application number: **06834436.5**

(22) Date of filing: **11.12.2006**

(86) International application number:
**PCT/JP2006/324681**

(87) International publication number:
**WO 2007/099681 (07.09.2007 Gazette 2007/36)**

(54) **LIVING BODY OBSERVATION APPARATUS**

AUSRÜSTUNG ZUR BEOBACHTUNG DES LEBENDEN KÖRPERS

EQUIPEMENT D'OBSERVATION DE CORPS VIVANT

(84) Designated Contracting States:
**DE FR GB**

(30) Priority: **03.03.2006 JP 2006058711**

(43) Date of publication of application:
**19.11.2008 Bulletin 2008/47**

(73) Proprietor: **Olympus Medical Systems Corp.**
**Tokyo 151-0072 (JP)**

(72) Inventors:
 • **YAMAZAKI, Kenji**
  **Tokyo 151-0072 (JP)**
 • **GONO, Kazuhiro**
  **Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
| | |
|---|---|
| **EP-A1- 1 576 920** | **JP-A- 4 183 430** |
| **JP-A- 03 105 483** | **JP-A- 08 313 823** |
| **JP-A- 2000 148 987** | **JP-A- 2002 034 893** |
| **JP-A- 2002 034 908** | **JP-A- 2003 093 336** |
| **JP-A- 2004 202 217** | **JP-A- 2005 141 659** |
| **JP-A- 2005 293 214** | **US-A1- 2003 139 650** |

**Description**

Technical Field

The present invention relates to a living body observation apparatus such as an endoscope apparatus which observes a living mucous membrane, e.g., in a body cavity.

Background Art

Endoscope apparatuses having an endoscope, a light source device, and the like have conventionally been in wide use in the medical field, etc.

In the medical field, for example, there are present normal observation for irradiating a subject such as a mucous membrane in a living body with white light and picking up an image of the subject which is substantially similar to observation with a naked eye, as well as narrow band light observation (NBI: Narrow Band Imaging) which is capable of picking up an image of a blood vessel of a mucous membrane superficial layer in a living body with better contrast than normal observation by irradiating the subject with narrow band light which is a light having a narrower band than irradiation light in normal observation and observing the subject is available to an endoscope apparatus. A first conventional example of an apparatus which performs the narrow band imaging is Japanese Patent Application Laid-Open Publication No. 2002-095635.

To obtain narrow band light used in the narrow band imaging described above, a band of illumination light needs to be narrowed. For this reason, it is necessary to narrow the illumination light by, e.g., inserting a filter to the broadband illumination light used in normal observation.

In contrast, Japanese Patent Application Laid-Open Publication No. 2003-93336 as a second conventional example discloses a narrow band light endoscope apparatus which obtains tissue information at a desired depth of a living tissue by conducting a signal processing on an image signal obtained using the normal illumination light thus generating a discrete spectral image.

The above-described second conventional example suffers from a drawback which complicates configuration in, e.g., that it is necessary to perform light amount control such that an image pickup signal is not saturated, in addition to estimation processing for obtaining a spectral image.

EP 1 576 920 A1 discloses an image processing circuit comprising a spectrum estimation unit for receiving each element of image data and obtaining data from en estimating data supply unit, which are then used for estimating a spectrum to estimate the spectrum of each pixel. The circuit further comprises a scattering feature calculation unit for calculating several scattering features based on the spectrum of each pixel from the spectrum estimation unit and based on data necessary for calculating features from a feature calculating data supply unit. The circuit also includes a color image generation unit for calculating display colors based on a scattering feature image from the scattering feature calculation unit and for determining the RGB values of each pixel, in order to display scattering features as a color image so as to output an RGB image.

JP 4 183430A discloses an image processing device for an endoscope. An original image is resolved into color signals R, G and B, before being inputted to an image processing device 104 and stored in memory. In addition, each of the R, G and B images is subjected to two-dimensional discrete Fourier transformation, and resolved into spatial frequency components. Furthermore, each image resolved into the spatial frequency components is subjected to a filtering process by setting a filter function having noise restriction capability in the way of spatial frequency, or a filter function having highlighting capability.

JP 2005 293214 A discloses a simulation image forming method for skin. The simulation image is formed by acquiring interior reflected light images and surface reflected light images of the skin of a subject, separating the interior reflected light images into melanin component by independent components analysis, resolving the melanin component interior reflected light image into a plurality of spatial frequency bands, selectively changing contrast with respect to the melanin component interior reflected light image resolved into the plurality of spatial frequency bands, synthesizing the melanin component interior reflected light images of the plurality of spatial frequency bands to form a melanin component adjusted interior reflected light image, forming a synthesized interior reflected light image by synthesizing the melanin component adjusted interior reflected light image and the interior reflected light images other than the melanin component, and changing irregular colors of the skin by synthesizing the synthesized interior reflected light images and the surface reflected light images.

US 2003/139650 A1 discloses an endoscope that includes a light source unit for illuminating an object, and an optical system which forms images of the object and includes a spectral filter. The spectral filter includes a first region which has a first spectral transmission and a second region which is peripheral to the first region which has a first spectral transmission that is different from the first spectral transmission, to thereby enable endoscope images of the object to be obtained in which fine details as carried high spatial frequencies in the image light of certain wavelengths are emphasized for those wavelengths that are passed by the second region of the spectral filter. In addition, the endoscope may contain a phase mask and an image processing means which serve to extend the depth of field in the wavelength range passed by the second region of the spectral filter.

The present invention has been made in consideration of the above-described points, and has as its object to provide a living body observation apparatus which generates image signals of an image allowing easy identification of living body structures such as mucous membrane structures on a superficial layer side and a deep layer side with a simple configuration.

Disclosure of Invention

Means for Solving the Problem

A living body observation apparatus according to the present invention is disclosed in claim 1.

Brief Description of the Drawings

Fig. 1 is a block diagram showing an overall configuration of an endoscope apparatus according to a first embodiment of the present invention;
Fig. 2 is a view showing a configuration of a rotating filter;
Fig. 3 is a graph showing spectral characteristics of R, G, and B filters provided at the rotating filter;
Fig. 4 is a block diagram showing a configuration of a filter circuit and surroundings;
Fig. 5 is a graph showing a frequency characteristic of a BPF constituting the filter circuit;
Fig. 6 is a graph showing a frequency characteristic of an HPF constituting the filter circuit;
Fig. 7 is a graph showing input-output characteristics of a γ correction circuit set in a second observation mode;
Fig. 8 is a graph for explaining working when the BPF in Fig. 5 is used;
Fig. 9 is a graph for explaining working when the HPF in Fig. 6 is used;
Fig. 10 is a block diagram showing an overall configuration of an endoscope apparatus according to a second embodiment of the present invention;
Fig. 11 is a block diagram showing a configuration of a wavelet transform portion according to a third embodiment of the present invention;
Fig. 12 is a chart showing an example of a configuration of transform coefficients of decomposition level 2 obtained by a two-dimensional discrete wavelet transform; and
Fig. 13 is a block diagram showing a configuration of a wavelet transform portion according to a modification.

Best Mode for Carrying Out the Invention

Embodiments of the present invention will be described below with reference to the drawings.

(First Embodiment)

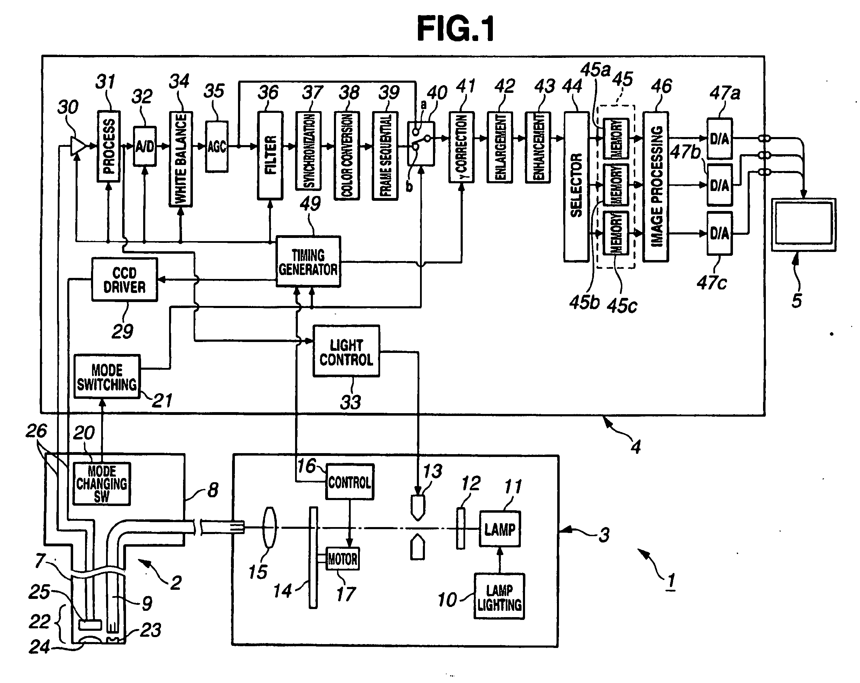

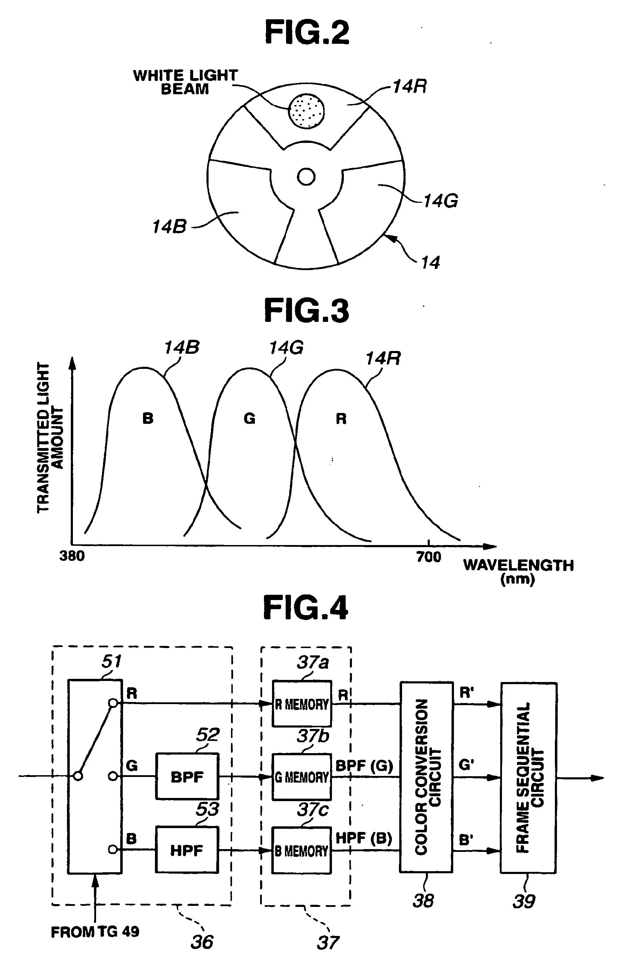

Figs. 1 to 9 relate to a first embodiment of the present invention. Fig. 1 is a diagram showing an overall configuration of an endoscope apparatus according to the first embodiment of the present invention. Fig. 2 is a view showing a configuration of a rotating filter. Fig. 3 is a graph showing spectral characteristics of R, G, and B filters provided at the rotating filter. Fig. 4 is a diagram showing a configuration of a filter circuit and surroundings.

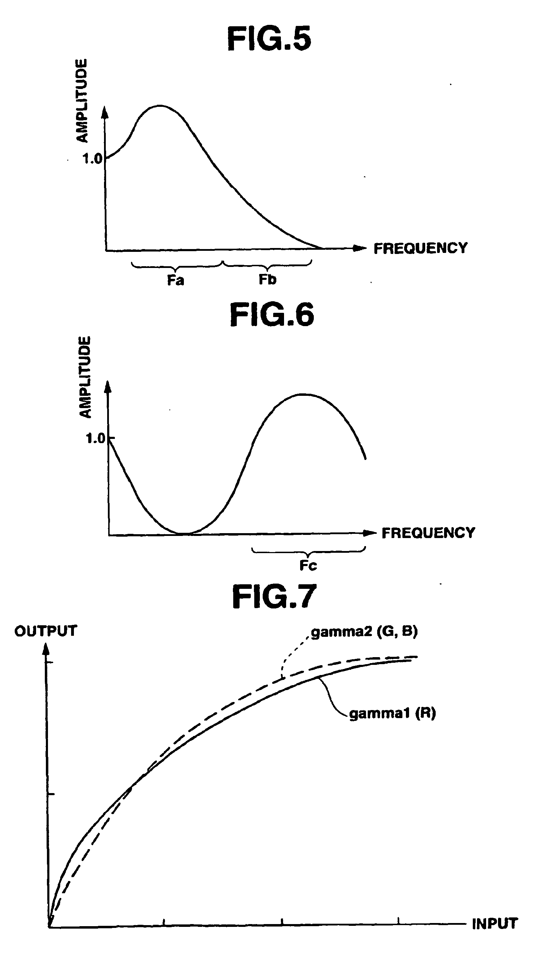

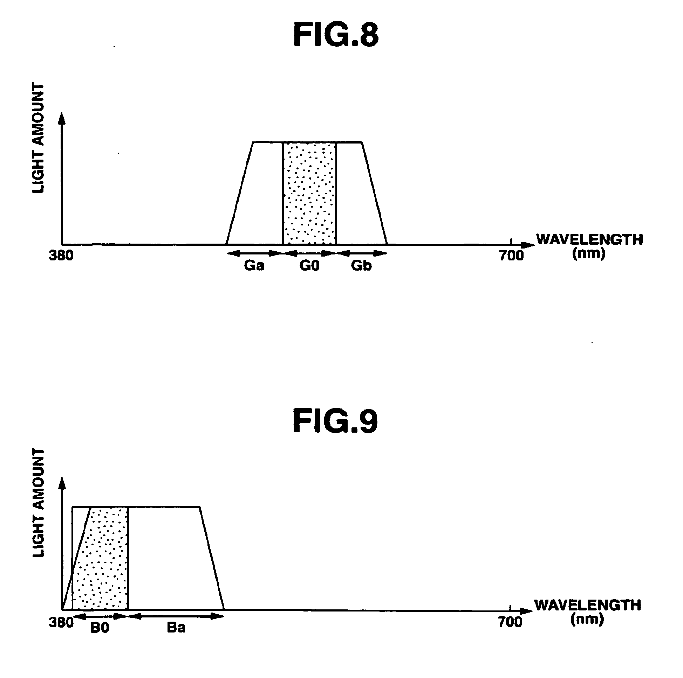

Fig. 5 is a graph showing a frequency characteristic of a BPF constituting the filter circuit. Fig. 6 is a graph showing a frequency characteristic of an HPF constituting the filter circuit. Fig. 7 is a graph showing input-output characteristics of a γ correction circuit set in a second observation mode. Fig. 8 is a graph for explaining working when the BPF in Fig. 5 is used. Fig. 9 is a graph for explaining working when the HPF in Fig. 6 is used.

As shown in Fig. 1, an endoscope apparatus 1 as the first embodiment of a living body observation apparatus according to the present invention includes: an electronic endoscope 2 which is inserted into a body cavity and picks up an image of a subject such as a living tissue and outputs the image as an image pickup signal in the body cavity; a light source device 3 for supplying the electronic endoscope 2 with a broadband illumination light for illuminating the subject side; a video processor 4 as signal processing means for generating a video signal as an image signal (also referred to as a biomedical signal) by driving image pickup means incorporated in the electronic endoscope 2 and performing a signal processing on an image pickup signal outputted from the electronic endoscope 2; and a monitor 5 as a display device which displays an image of a subject on the basis of a video signal outputted from the video processor 4.

The electronic endoscope 2 has an elongated insertion portion 7 to be inserted into a body cavity, and an operation portion 8 is provided at a rear end of the insertion portion 7. A light guide 9 for transmitting illumination light is inserted in the insertion portion 7, and a rear end (proximal end) of the light guide 9 is detachably connected to the light source device 3.

The light source device 3 includes a lamp 11 such as a xenon lamp which generates broadband illumination light covering a visible region upon supply of lighting power from a lamp lighting circuit 10, a heat wave cut filter 12 which cuts off a heat wave in illumination light, an aperture device 13 which limits the amount of illumination light having passed through the heat wave filter 12, a rotating filter 14 which converts illumination light into frame sequential light, a condenser lens 15 which condenses and supplies frame sequential light having passed through the rotating filter 14 on an incident surface of the light guide 9 disposed in the electronic endoscope 2, and a control circuit 16 which controls rotation of the rotating filter 14.

As shown in Fig. 2, the rotating filter 14 is provided with three filters, an R filter 14R, a G filter 14G, and a B filter 14B, which transmit, over a broad band, lights of red (R), green (G), and blue (B) wavelengths, respectively, which are arranged in a fan-shape in a circumferential direction of a disk.

Fig. 3 is a view showing spectral transmission characteristics of the R filter 14R, G filter 14G, and B filter 14B. The R filter 14R, G filter 14G, and B filter 14B have the properties to transmit lights of the R, G, and B wavelength ranges, respectively, over a broad band.

The rotating filter 14 is rotated at a predetermined rotational speed by a motor 17 which is drive-controlled by the control circuit 16. The rotating filter 14 is rotated to sequentially place the R filter 14R, G filter 14G, and B filter 14B in an illumination optical path, so that the R, G, and B lights are sequentially condensed and entered on the incident surface of the light guide 9 by the condenser lens 15.

The illumination light transmitted by the light guide 9 is irradiated onto a body cavity tissue side through an illumination lens 23 which is attached to an illumination window of a distal end portion 22 of the insertion portion 7.

An objective lens 24 is attached to an observation window which is provided adjacent to the illumination window. At an image formation position of the objective lens 24, a charge coupled device (hereinafter abbreviated as a CCD) 25 is placed as an image pickup device. The CCD 25 photoelectrically converts an optical image formed by the objective lens 24.

The CCD 25 is connected to a CCD driver 29 and a preamp 30 in the video processor 4 through signal lines 26. Note that the signal lines 26 are actually detachably connected to the video processor 4 through connectors (not shown).

The CCD 25 is driven applied with a CCD drive signal from the CCD driver 29. An image pickup signal obtained from photoelectric conversion is amplified by the preamp 30 and then inputted to an A/D converter circuit 32 and a light control circuit 33 through a process circuit 31 which performs correlated double sampling (CDS), noise removal, and the like.

The image pickup signal is converted from an analog signal into a digital signal by the A/D converter circuit 32, the digital signal is subjected to a white balance processing by a white balance circuit 34. The resulting signal is then amplified to a predetermined level by an automatic gain control circuit (hereinafter abbreviated as an AGC circuit) 35.

Note that, in the present embodiment, dimming operation on the amount of illumination light by the aperture device 13 of the light source device 3 is performed in preference to operation of the AGC circuit 35 and that, after an aperture of the aperture device 13 reaches an open state, the AGC circuit 35 performs an operation of amplifying the signal on the basis of information on the open state to increase an insufficient signal level.

The light control circuit 33 generates, based on the output signal from the process circuit 31, a light control signal for controlling the amount of illumination light to an appropriate amount by adjusting an aperture value of the aperture device 13 of the light source device 3.

Data outputted from the above-described AGC circuit 35 is inputted to a filter circuit 36 forming separation means in the present embodiment and to a γ correction circuit 41 through a selector switch 40.

The electronic endoscope 2 is provided with a mode changing switch 20 which is operated by a surgeon or the like to allow selected observation between, e.g., two observation modes, a first observation mode serving as a normal observation mode and a second observation mode serving as a living mucous membrane-enhanced observation mode for enhanced observation of a structure of a living mucous membrane.

An instruction to switch between the observation modes given by the mode changing switch 20 is inputted to a mode switching circuit 21 of the video processor 4. In response to the observation mode switching instruction, the mode switching circuit 21 flips the selector switch 40 and sends a mode switching signal to a timing generator 49.

Note that the mode changing switch 20 may not be provided in the electronic endoscope 2. For example, the mode changing switch 20 may be provided at a front panel (not shown) of the video processor 4 or may be configured as a predetermined key of a keyboard (not shown) connectable to the video processor 4.

In response to the operation of the mode changing switch 20, the selector switch 40 selects a contact a in the first observation mode corresponding to normal observation and a contact b in the second observation mode, on the basis of the observation mode switching instruction outputted via the mode switching circuit 21.

Thus, if the second observation mode is selected, an output signal from the AGC circuit 35 is passed through the filter circuit 36, processed by a synchronization circuit 37, a color conversion circuit 38, and a frame sequential circuit 39, and then inputted to the γ correction circuit 41 through the selector switch 40 The filter circuit 36 here serves as separation means for separating and extracting main structures of a living body serving as an object to be observed, more particularly spatial frequency components of a fine mucous membrane structure and a coarse mucous membrane structure.

The filter circuit 36 includes a selector 51 which is flipped by a timing signal from the timing generator 49 and a band-pass filter (hereinafter abbreviated as a BPF) 52 and a high-pass filter (hereinafter abbreviated as an HPF) 53 with set frequency characteristics which allow separation and extraction of spatial frequency components corresponding to main mucous membrane structures of a living body, as shown in Fig. 4.

As shown in Fig. 4, the selector 51 is flipped by the timing generator 49 at the timing when broadband R, G, and B signals are each inputted to the filter circuit 36 in a frame sequential manner.

An R signal is stored as-is in an R memory 37a of the synchronization circuit 37, a G signal is stored in a G memory 37b through the BPF 52, and a B signal is stored in a B memory 37c through the HPF 53.

That is, an R signal is directly stored in the R memory 37a, a G signal is filter-processed by the BPF 52 and is stored in the G memory 37b, and a B signal is filter-processed by the HPF 53 and is stored in the B memory 37c.

In this case, the BPF 52 is set to have a filter characteristic (frequency characteristic) which amplifies a frequency component in a middle or a low and middle frequency band Fa such that an amplitude of the frequency component is larger than 1 and suppresses a high frequency band Fb, as shown in Fig. 5. The HPF 53 is set to have a filter characteristic which amplifies a frequency component in a high frequency band Fc such that an amplitude of the frequency component is larger than 1, as shown in Fig. 6. Note that the BPF 52 and the HPF 53 are set so as not to change the value of a DC component. Specifically, the BPF 52 and the HPF 53 are set such that the amplitude of a DC component is 1.

The filter circuit 36 constituting the separation means in the present embodiment separates a fine mucous membrane structure and a coarse mucous membrane structure in a living body from each other. In order to facilitate identification of the structures, they are subjected to contrast conversion processing in the γ correction circuit 41

Pieces of R, G, and B signal data respectively stored in the R, G, and B memories 37a, 37b, and 37c of the above-described synchronization circuit 37 are simultaneously read out to produce synchronized R, G, and B signals. The R, G, and B signals are inputted to the color conversion circuit 38 serving as color adjustment means and are color-converted. Note that since the G and B signals have undergone filter processes by the BPF 52 and HPF 53, respectively, as shown in Fig. 4, the G and B signals are denoted by BPF(G) and HPF(B).

The color conversion circuit 38 color-converts synchronized pieces of image information, R, BPF(G), and HPF (B), using a 3 x 3 matrix. The pieces of image information are subjected to color conversion processing such that a fine structural portion on a superficial layer side and a coarse structural portion on a deep layer side of a mucous membrane are displayed in different tones. Such color conversion processing causes separated mucous membrane structures to be displayed in different tones, better facilitating identification of the fine structural portion on a superficial layer side of a mucous membrane and a coarse structural portion on a deep layer side thereof.

A conversion equation for color conversion from R, BPF(G), and HPF(B) into R', G', and B' in this case is given by the following formula 1 using a 3 x 3 matrix K:

[Formula 1]

$$\begin{pmatrix} R' \\ G' \\ B' \end{pmatrix} = K \begin{pmatrix} R \\ BPF(G) \\ HPF(B) \end{pmatrix} \quad \text{Formula (1)}$$

$$K = \begin{pmatrix} 0 & m1 & 0 \\ 0 & 0 & m2 \\ 0 & 0 & m3 \end{pmatrix}$$

The matrix K is composed of, e.g., three real elements m1 to m3 (the other elements are 0). Use of a conversion equation like Formula 1 increases weights (ratios) of the BPF(G) and HPF(B) color signals of the R, BPF(G), and HPF(B) color signals. In the example, the R color signal having a long wavelength is suppressed.

Output signals from the color conversion circuit 38 (although the signals have been converted into signals denoted by R', G', and B', a following description will be given using R, G, and B for sake of simplicity except for confusing cases) are inputted to the frame sequential circuit 39.

The frame sequential circuit 39 is composed of frame memories. The frame sequential circuit 39 sequentially reads out the simultaneously stored R, G, and B image data as color component images, thereby converting the color components images into pieces of frame sequential image data. The frame sequential R, G, and B image data are passed through the selector switch 40 and then subjected to γ correction by the γ correction circuit 41.

The γ correction circuit 41 includes inside thereof, e.g., a γ table storing input-output characteristics for γ correction, which is switched over by the timing generator 49.

In the first observation mode, the γ correction circuit 41 is set to have an input-output characteristic for performing common γ correction on R, G, and B signals inputted in a frame sequential manner. In the second observation mode, the input-output characteristics for γ correction is switched over for each of R, G, and B signals inputted in a frame sequential manner.

More specifically, the γ correction circuit 41 performs a contrast conversion processing as follows. For an R signal, the γ correction circuit 41 is set to have a gamma1 input-output characteristic indicated by a solid line in Fig. 7. For G and B signals which reproduce fine structure information of a mucous membrane superficial layer better than the R signal, the γ correction circuit 41 is set to have a gamma2 input-output characteristic indicated by a dotted line in Fig. 7.

The gamma2 input-output characteristic is set to have a smaller output than the gamma1 input-output characteristic in a range of small inputs and have a larger output than the gamma1 input-output characteristic in a range of large inputs.

The γ correction circuit 41 performs γ correction on G and B signals with an input-output characteristic as described above, thereby allowing enhancement in contrast of fine mucous membrane structure information to be reproduced by image signals.

Those signals having undergone the γ correction by the γ correction circuit 41 are subjected to enlargement interpolation processing by an enlargement circuit 42 and then inputted to an enhancement circuit 43.

Those signals processed by the enlargement circuit 42 are each subjected to structure enhancement or edge enhancement by the enhancement circuit 43 and then inputted to a synchronization circuit 45 through a selector 44. The synchronization circuit 45 is formed of three memories 45a, 45b, and 45c.

The R, G, and B image data synchronized by the synchronization circuit 45 are inputted to an image processing circuit 46 to undergo an image processing such as moving image color shift correction, and then inputted to D/A converter circuits 47a, 47b, and 47c. The R, G, and B image data inputted to the D/A converter circuits 47a, 47b and 47c are converted into analog video signals or image signals

(biomedical signals in a broad sense) by the D/A converter circuits 47a, 47b and 47c, and then inputted to the monitor 5 as a display device. The monitor 5 displays an endoscope image corresponding to inputted video signals. The timing generator 49 is provided in the video processor 4. The timing generator 49 is inputted with a synchronization signal in synchronism with rotation of the rotating filter 14 from the control circuit 16 of the light source device 3. In response, the timing generator 49 outputs various types of timing signals in synchronism with the synchronization signal to the above-described circuits.

The light control circuit 33 controls the aperture device 13 of the light source device 3, thereby controlling the illumination light amount so as to obtain an image with an appropriate brightness suitable for observation.

Working of the endoscope apparatus 1 according to the present embodiment with the above-described configuration will now be described.

First, a surgeon or the like connects the electronic endoscope 2 to the light source device 3 and video processor 4, as shown in Fig. 1, and turns on power. The surgeon or the like inserts the electronic endoscope 2 into a body cavity and observes a living tissue of a part to be observed in the body cavity. The portions of the endoscope apparatus 1 are initially set for, e.g., the first observation mode as normal observation.

When the rotating filter 14 is rotated at a constant speed in an optical path of illumination light, the R, G, and B illumination lights are condensed by the condenser lens 15 and come incident on the light guide 9. Broadband R, G, and B illumination lights as shown in Fig. 3 are irradiated from a distal end surface of the light guide 9, passing through the illumination lens 23, and sequentially applied to the living tissue.

The CCD 25 picks up a living tissue image under the broadband R, G, and B illumination lights. The CCD 25 photoelectrically converts the picked up image, and the resulting CCD output signals are amplified by the preamp 30 in the video processor 4. A CDS circuit in the process circuit 31 then extracts signal components from the CCD output signals. Output signals from the process circuit 31 are converted into digital signals by the A/D converter circuit 32. The digital signals pass through the white balance circuit 34 and AGC circuit 35 (in the first observation mode as described above) and then are inputted from the selector switch 40 to the γ correction circuit 41.

Output signals from the selector switch 40 are subjected to γ correction by the γ correction circuit 41, to enlargement interpolation processing by the enlargement circuit 42, and then to structure enhancement or edge enhancement by the enhancement circuit 43. The resulting signals are thereafter inputted to the synchronization circuit 45 through the selector 44. Pieces of image data synchronized by the synchronization circuit 45 are subjected to image processes such as moving image color shift correction by the image processing circuit 46. The processed data are next converted into analog video signals by the D/A converter circuits 47a, 47b, and 47c and then outputted to the monitor 5. The monitor 5 displays an endoscope image corresponding to the inputted video signals.

As a result, in the first observation mode, a normal color image formed through illumination light in a visible region is displayed on the monitor 5.

Meanwhile, if the mode changing switch 20 of the electronic endoscope 2 is operated to give an instruction to switch to the second observation mode, a signal according to the switching instruction is inputted to the mode switching circuit 21 of the video processor 4.

The mode switching circuit 21 sends to the timing generator 49 a mode switching signal acknowledging completion of the switching instruction to the second observation mode and flips the selector switch 40 such that the contact b is ON.

As shown in Fig. 4, the timing generator 49 sequentially flips the selector 51 at a time when broadband R, G, and B signals are each inputted to the filter circuit 36. In this case, an R signal passes through the filter circuit 36 without being filter-processed and is stored in the R memory 37a of the synchronization circuit 37.

In contrast, a frequency component in the low and middle frequency band Fa is extracted (separated) from the G signal by the BPF 52 set to have a frequency characteristic as shown in Fig. 5 which suppresses the high frequency band Fb and amplifies the low and middle frequency band Fa.

Furthermore, a frequency component in the high frequency band Fc is extracted (separated) from the B signal by the HPF 53 set to have a characteristic as shown in Fig. 6 which amplifies the high frequency band Fc.

Thus, the BPF 52 and HPF 53 of the filter circuit 36 are set to have frequency separation characteristics for separating and extracting spatial frequency components corresponding to a structure on a superficial layer side and a structure on a side located deeper than the superficial layer of a living mucous membrane (e.g., bloodstream structures) and characteristics for allowing easy identification of the structures generate signals. The BPF 52 and HPF 53 generate a signal which increases visibility of the structures, as described below.

Fig. 8 is a graph for explaining separation and extraction of a G signal component similar to a G signal obtained by image pickup under narrow band G illumination light using the BPF 52 in Fig. 5.

The trapezoid in Fig. 8 represents broadband G illumination light. The near-center band of the G illumination light is limited so as to include a wavelength range G0 suitable for obtaining a coarse mucous membrane structure, a short wavelength range Ga nearer to a short wavelength side than the wavelength range G0, and a long wavelength range Gb nearer to a long wavelength side than the wavelength range G0. Absorbance of hemoglobin is low in the short wavelength range Ga. The contrast of a blood vessel figure or the like becomes low compared to the wavelength range G0 in a G signal obtained from image pickup by the CCD 25, while contributing to generation of an image representing a fine mucous membrane structure of a shallow layer (superficial layer).

This fine mucous membrane structure appears as high-frequency components. Therefore, setting the characteristic of the BPF 52 to one which suppresses a high frequency side suppresses reproduction of the fine mucous membrane structure.

Although the long wavelength range Gb reproduces information of a deeper layer than the wavelength range G0, much of the information is for a structure of a large blood vessel at a deep layer. It is thus conceivable that the information is not much different from information reproduced by the adjacent wavelength range G0. Rather, the long wavelength range Gb has a lower hemoglobin absorbance and hence a lower contrast than the wavelength range G0. Accordingly, when image information of the long wavelength range Gb and high-contrast image information reproduced by the wavelength range G0 are superimposed to each other and averaged, overall contrast is decreased.

For this reason, if a frequency characteristic of the BPF 52 is set to a frequency characteristic as shown in Fig. 5 which enhances contrast of a frequency component in the low and middle ranges, a signal as a frequency component in the low and middle ranges can be amplifyingly extracted. Accordingly, G signal components corresponding to an image of a coarse mucous membrane structure on a deep layer side are obtained.

Fig. 9 is a graph for explaining extraction of a B signal component similar to a B signal obtained by image pickup under narrow band B illumination light using the HPF 53 in Fig. 6.

The trapezoid in Fig. 9 represents broadband B illumination light. The B illumination light is band-limited to a narrow band and includes a wavelength range B0 suitable for obtaining a fine mucous membrane structure and a long wavelength range Ba nearer to a long wavelength side than the wavelength range B0. Since the long wavelength range Ba has longer wavelengths than the wavelength range B0, the long wavelength range Ba contributes to reproducing information of a mucous membrane slightly deeper than the wavelength range B0.

B image data obtained from the long wavelength range Ba serves as middle frequency components and also serves as an object to be suppressed. For this reason, a frequency characteristic of the HPF 53 is set to a characteristic which suppresses the band, as shown in Fig. 6.

While contributing to reproducing the same mucous membrane information as the wavelength range B0, the long wavelength range Ba has a lowered contrast than in the wavelength range B0 due to the low hemoglobin absorbance. That is, an image, in which the long wavelength range Ba and the wavelength range B0 with high contrast are averaged, has a lower contrast than an image applied with only the wavelength range B0.

For this reason, in the present embodiment, applying the HPF 53 to an image pickup signal results in a frequency characteristic with an amplified high frequency band, thereby enhancing the contrast in the high frequency band. In the above-described manner, a B image in which a fine mucous membrane structure on a superficial layer side is easily-viewable can be generated.

As described above, G and B signals representing mucous membrane structures similar to narrow band G and B signals are synchronized together with an R signal. After that, the signals are color-converted by the color conversion circuit 38 to have tones which make the mucous membrane structures more easily-identifiable. Output signals from the color conversion circuit 38 are further converted into frame sequential signals. Then, in the γ correction circuit 41, the G and B signals are subjected to contrast conversion processing for amplifying a difference between outputs in a small input range and a large input range. Thus, an image with easy visual recognition of a mucous membrane structure on a superficial layer side is displayed on the monitor 5.

As such, the image displayed on the monitor 5 is represented as an image facilitating identification of a fine mucous membrane structural portion and a coarse mucous membrane structural portion in a living body, in that these portions are separated from each other on the basis of frequency characteristics corresponding to spatial frequencies of the structural portions.

For this reason, according to the present embodiment, it is possible to observe a fine mucous membrane structural portion and a coarse mucous membrane structural portion in a living body as an easily-identifiable image with a simple configuration. The present embodiment thus has an effect of providing an image allowing easy diagnosis.

(Second Embodiment)

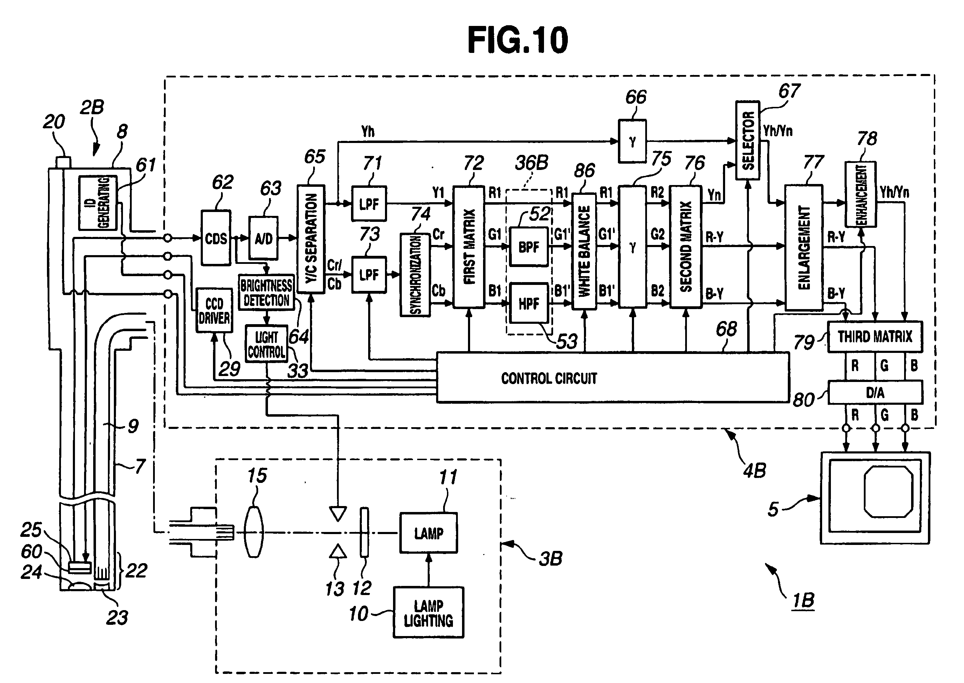

A second embodiment of the present invention will be described with reference to Fig. 10. Fig. 10 shows an endoscope apparatus 1B according to the second embodiment of the present invention. While the endoscope apparatus I of the first embodiment is a frame sequential type endoscope apparatus, the endoscope apparatus 1B of a simultaneous type is used in the present embodiment.

The endoscope apparatus 1B includes an electronic endoscope 2B, a light source device 3B, a video processor 4B, and a monitor 5.

The electronic endoscope 2B is formed by attaching, as a color separation filter 60 which performs optical color separation, complementary filters for respective pixels to an image pickup surface of the CCD 25 in the electronic endoscope 2 shown in Fig. 1. Although not shown, the complementary filters are four color chips of magenta (Mg), green (G), cyan (Cy), and yellow (Ye) arranged in front of each pixel. Specifically, in a horizontal direction of the complementary filters, Mg and G color chips are alternately arranged. In a vertical direction of the complementary filters, an array of Mg, Cy, Mg and Ye color chips and an array of G, Ye, G and Cy color chips are arranged in that arrangement order.

The CCD 25 using the complementary filters is configured such, when pixels at two rows adjacent in the vertical direction are added and are sequentially read out, pixel rows for odd-numbered fields and pixel rows for even-numbered fields are staggered. As is publicly known, luminance signals and color difference signals can be generated by the color separation circuit on a downstream side.

In, e.g., an operation portion 8 in the electronic endoscope 2B, an ID generating circuit 61 is provided. The ID information of the ID generating circuit 61 can be used to change the characteristic for signal processing depending on, e.g., the type of the color separation filter 60 of the CCD 25 in the electronic endoscope 2B and variation between the color separation filters 60, thereby performing a more appropriate signal processing.

The light source device 3B has a configuration of the light source device 3 in Fig. 1 excluding the rotating filter 14, motor 17, and control circuit 16.

That is, the light source device 3B condenses white illumination light by a condenser lens 15 and brings the white illumination light incident on a proximal end surface of a light guide 9. The illumination light passes from a distal end surface of the light guide 9 through an illumination lens 23 and is then irradiated onto a living tissue of a part to be observed in a body cavity. An optical image of the illuminated living tissue is separated into complementary colors by the color separation filter 60 and is picked up by the CCD 25.

An output signal from the CCD 25 is inputted to a CDS circuit 62 in the video processor 4B. The CDS circuit 62 extracts a signal component from the output signal from the CCD 25 and converts them into a baseband signal. The baseband signal is then converted into a digital signal by an A/D converter circuit 63, and at the same time has its brightness (average luminance of the signal) detected by a brightness detection circuit 64.

The signal having the brightness detected by the brightness detection circuit 64 is inputted to a light control circuit 33, and a light control signal for dimming using a difference from reference brightness (a target value for dimming) is generated. The light control signal from the light control circuit 33 is used to control an aperture device 13 of the light source device 3B, thus adjusting the light to obtain an illumination light amount suitable for observation.

The digital signal outputted from the A/D converter circuit 63 is processed by a Y/C separation circuit 65 to be a luminance signal Y and line sequential color difference signals Cr (= 2R - G) and Cb (= 2B - G) (as color signals C in a broad sense). The luminance signal Y is inputted to a selector 67 through a γ circuit 66 (the luminance signal will be denoted by Yh hereinafter) and to an LPF 71 which limits a signal passband.

The LPF 71 is set to have a broad passband for the luminance signal Y. A luminance signal Y1 having a band set by a passband characteristic of the LPF 71 is inputted to a first matrix circuit 72.

The color difference signals Cr and Cb are inputted to a synchronization circuit 74 (in a line sequential manner) through a second LPF 73 which limits a signal passband.

In this case, a passband characteristic of the second LPF 73 is changed by a control circuit 68 depending on an observation mode. More specifically, the second LPF 73 is set to have a passband lower than the first LPF 71 (low band) in a first observation mode corresponding to normal observation.

On the other hand, the second LPF 73 is changed to have a broader band than the low band in the first observation mode for normal observation, in a second observation mode for mucous membrane-enhanced observation. For example, the second LPF 73 is set (changed) to have a broadband in much a same manner as the first LPF 71. As described above, the second LPF 73 changes the passband for the color difference signals Cr and Cb in conjunction with switching between the observation modes. Note that a change of the characteristic of the second LPF 73 in conjunction with switching between the observation modes is performed under control of the control circuit 68.

The synchronization circuit 74 produces the synchronized color difference signals Cr and Cb, which are then inputted to the first matrix circuit 72.

The first matrix circuit 72 converts the luminance signal Y and color difference signals Cr and Cb into R1, G1, and B1 color signals.

The first matrix circuit 72 is controlled by the control circuit 68. The first matrix circuit 72 changes a value of a matrix coefficient (which determines a conversion characteristic) depending on a characteristic of the color separation filter 60 of the CCD 25, thereby converting the luminance signal Y and color difference signals Cr and Cb into the R1, G1, and B1 color signals free or almost free of a mixed color.

For example, the characteristic of the color separation filter 60 of the CCD 25 mounted at the electronic endoscope 2B may vary according to the electronic endoscope 2B actually connected to the video processor 4B. In this case, the control circuit 68 changes the coefficient of the first matrix circuit 72 using the ID information depending on the characteristic of the color separation filter 60 of the actually used CCD 25.

With the above-described configuration, it is possible to appropriately cope with a case where the type of the actually used image pickup means is different, prevent generation of a false color, and make a conversion into the three R1, G1, and B1 primary color signals free of a mixed color.

The R1, G1, and B1 color signals generated by the first matrix circuit 72 are outputted to a white balance circuit 86 through a filter circuit 36B corresponding to the filter circuit 36 in the first embodiment.

In the first embodiment, since frame sequential R, G, and B signals are inputted to the filter circuit 36, the selector 51 as shown in Fig. 4 is used. In the present embodiment in contrast, since the R1, G1, and B1 color signals are simultaneously inputted, the selector 51 in Fig. 4 is unnecessary.

The R1 signal passes through the filter circuit 36B without being filtered and is inputted to the white balance circuit 86. The G1 and B 1 signals turn into G1' and B1' signals, respectively, through the BPF 52 and HPF 53 and are then inputted to the white balance circuit 86.

In the present embodiment, the filter circuit 36B performs substantially the same signal processing as in the first embodiment. The white balance circuit 86, to which the R1 signal and G1' and B1' signals having passed through the filter circuit 36B are inputted, and a γ circuit 75 to which signals outputted from the white balance circuit 86 are inputted are controlled by the control circuit 68.

The white balance circuit 86 performs white balance adjustment on the inputted R1, G1', and B1' signals and outputs the R1, G1', and B1' signals after the white balance adjustment to the γ circuit 75.

Also in the present embodiment, an image pickup signal is subjected by the γ circuit 75 to a contrast conversion processing which is basically the same as the γ correction circuit 41 in the first embodiment. That is, in the first observation mode, the R1, G1', and B1' signals are γ-corrected with a common input-output characteristic, while in the second observation mode, the R1, G1', and B1' signals are γ-corrected with different input-output characteristics.

While the γ correction in the γ correction circuit 41 is performed after color conversion in the first embodiment, the present embodiment is differently configured to perform color conversion in a second matrix circuit 76 (to be described later) after γ correction.

For this reason, in the present embodiment, the R1 and G1' signals are γ - corrected (simultaneously in this case) with the γ 1 input-output characteristic in Fig. 7, and the B1' signal is γ -corrected with the γ 2 input-output characteristic in Fig. 7, in the second observation mode.

As described above, signals are changed to have a γ characteristic with a more enhanced γ correction characteristic than in the first observation mode. In such a state, the γ circuit 75 of the present embodiment performs a contrast conversion processing. Thus, the present embodiment can provide a display allowing easier identification with an enhanced contrast.

R2, G2, and B2 color signals subjected to the γ correction by the γ circuit 75 are converted by the second matrix circuit 76 into a luminance signal Yn and color difference signals R-Y and B-Y.

In this case, the control circuit 68 sets a matrix coefficient of the second matrix circuit 76 such that, in the first observation mode, the second matrix circuit 76 simply converts the R2, G2, and B2 signals into the luminance signal Yn and color difference signals R-Y and B-Y

In the second observation mode, the control circuit 68 changes the matrix coefficient of the second matrix circuit 76 to a matrix coefficient which causes the second matrix circuit 76 to also perform color conversion to be performed by the color conversion circuit 38 in the first embodiment, i.e., also serve as color adjustment means.

The luminance signal Yn outputted from the second matrix circuit 76 is inputted to the selector 67. Switching in the selector 67 is controlled by the control circuit 68. That is, the luminance signal Yh is selected in the first observation mode while the luminance signal Yn is selected in the second observation mode.

The color difference signals R-Y and B-Y outputted from the second matrix circuit 76 are inputted to an enlargement circuit 77 together with the luminance signal Yh or Yn (hereinafter denoted as Yh/Yn) having passed through the selector 67.

The luminance signal Yh/Yn having undergone enlargement processing by the enlargement circuit 77 is subjected to edge enhancement by an enhancement circuit 78 and is then inputted to a third matrix circuit 79. The color difference signals R-Y and B-Y having undergone the enlargement processing by the enlargement circuit 77 are inputted to the third matrix circuit 79 without passing through the enhancement circuit 78.

The luminance signal Yh/Yn and color difference signals R-Y and B-Y are converted into three R, G, and B primary color signals by the third matrix circuit 79. The converted signals are further converted into three analog primary color signals by a D/A converter circuit 80 and then outputted from video signal output terminals to the monitor 5.

Note that, edge enhancement by the enhancement circuit 78 may also have its enhancement characteristic (whether an enhancement band is set to a low and middle band or a middle and high band) etc., changed depending on the types of the CCD 25, color separation filter 60, and the like.

The present embodiment with the above-described configuration basically acts as the separation processing on a signal picked up by the CCD 25 in a frame sequential manner using a spatial frequency component as described in the first embodiment, which is applied to the simultaneous type.

More specifically, the filter circuit 36 performs a process of performing separation by using spatial frequency components with respect to R, G, and B signals picked up in a frame sequential manner and sequentially inputted and the like in the first embodiment. In the present embodiment in contrast, the filter circuit 36B performs a process of performing separation of spatial frequency components from simultaneously inputted R, G, and B signals and the like.

Therefore, the present embodiment of the simultaneous type case can also achieve almost the same effects as in the first embodiment of the sequential type case.

That is, it is made possible to observe a fine mucous membrane structure on a superficial layer side and a coarse mucous membrane structure on a deep layer side of a living mucous membrane in an image in which the mucous membrane structures are easily-identifiable in a same illumination condition as normal observation, like image pickup under narrow band light.

Note that, in the above-described first and second embodiments, the filter circuits 36 and 36B perform frequency-based separation and perform contrast conversion processing in consideration of a reflection characteristic (light absorption characteristic) of a living mucous membrane. The present invention nevertheless also includes simple separation (extraction) of a spatial frequency intended to be separated from a living structure.

For example, the present invention includes separation into a biomedical signal corresponding to at least one of a fine mucous membrane structure on a superficial layer side and a coarse mucous membrane structure, by using an HPF or LPF having, as a cutoff frequency, a spatial frequency between spatial frequency components corresponding to the mucous membrane structures.

(Third Embodiment)

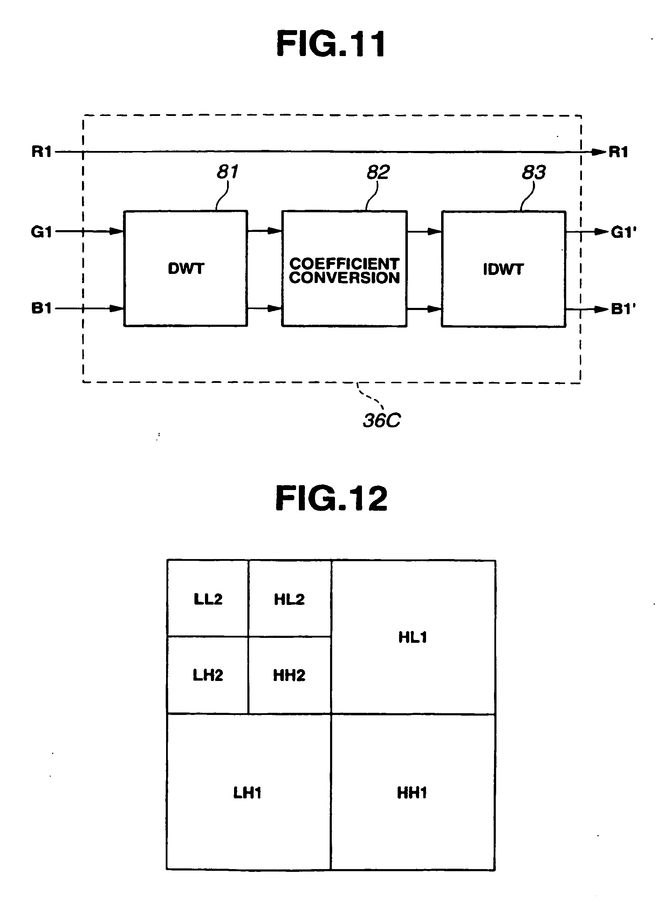

A third embodiment of the present invention will be described with reference to Figs. 11 to 13. Fig. 11 shows a wavelet transform portion 36C as separation means according to the third embodiment of the present invention. An endoscope apparatus of the present embodiment has a configuration in which the wavelet transform portion 36C shown in Fig. 11 is used instead of the filter circuit 36B in the endoscope apparatus 1B in Fig. 10.

As shown in Fig. 11, the wavelet transform portion 36C includes a wavelet transform circuit (hereinafter abbreviated as a DWT) 81 which performs a two-dimensional discrete wavelet transform on G1 and B1 signals shown in Fig. 10, a coefficient conversion circuit 82 which performs predetermined weighting processing on a wavelet transform coefficient outputted from the DWT 81, and an inverse wavelet transform circuit (hereinafter abbreviated as an IDWT) 83 which performs a two-dimensional inverse discrete wavelet transform on an output from the coefficient conversion circuit 82.

Note that an R signal passes through the wavelet transform portion 36C without being processed and is inputted to a first matrix circuit 72.

The DWT 81 performs a two-dimensional discrete wavelet transform using a Haar basis. The two-dimensional discrete wavelet transform uses a separable two-dimensional filter including two one-dimensional filters, one used for a horizontal direction and the other used for a vertical direction, and is publicly known, and a description of the two-dimensional discrete wavelet transform will be omitted.

Fig. 12 is an example of a configuration of transform coefficients of decomposition level 2 in a two-dimensional discrete wavelet transform by the DWT 81. In Fig. 12, transform coefficients (image components) divided into subbands by a discrete wavelet transform are denoted by HH1, LH1, HL1, HH2, LH2, HL2 and LL2.

For example, HH1 represents an image component obtained by using a high-pass filter both in the horizontal and vertical directions, and x in HHx represents a decomposition level of an original image.

Similarly, an image component LH represents one obtained by applying a low-pass filter in the horizontal direction and a high-pass filter in the vertical direction. An image component HL represents one obtained by applying a high-pass filter in the horizontal direction and a low-pass filter in the vertical direction. An image component LL represents one obtained by applying a low-pass filter in the horizontal direction and a low-pass filter in the vertical direction. The transform coefficients HH2, HL2, LH2, and LL2 are derived by decomposing the transform coefficient LL1 into subbands. Note that, at decomposition level 1, an image before decomposition is decomposed into four transform coefficients HH1, LH1, HL1, and LL 1.

The DWT 81 makes a decomposition level for an inputted G signal (as an original signal) lower than a decomposition level for a B signal. For example, the DWT 81 sets the decomposition level to 1 and decomposes a G signal into HH1, LH1, HL1, and LL1. On the other hand, the DWT 81 makes the decomposition level for an inputted B signal higher than the decomposition level for a G signal. For example, the DWT 81 sets the decomposition level to 4 and decomposes the B signal into HH1, LH1 and HL1, HH2, LH2 and HL2, HH3, LH3 and HL3, and HH4, LH4, HL4 and LL4.

Transform coefficients generated by the DWT 81 in the above-described manner are inputted to the coefficient conversion circuit 82.

In weighting by the coefficient conversion circuit 82, the transform coefficients HH1, LH1, and HL1 are multiplied by weighting factors for a G signal to be reduced.

For example, the weighting factors are uniformly set to O. This makes it possible to suppress high-frequency components in the horizontal direction, the vertical direction, and a diagonal direction. The transform coefficient LL1 is multiplied by 1 as a weighting factor.

On the other hand, the transform coefficients HH2, LH2 and HL2, HH3, LH3 and HL3, and HH4, LH4 and HL4 are multiplied by weighting factors for a B signal to be reduced. For example, the weighting factors are uniformly set to O. This suppresses frequency components in the low and middle frequency bands. The transform coefficients HH1, LH1, HL1, and LL4 are multiplied by weighting factors of 1.

Coefficients which have undergone weighting processing by the coefficient conversion circuit 82 and are outputted in the above-described manner are inputted to the IDWT 83 and then subjected to a two-dimensional inverse discrete wavelet transform.

In this case, a G signal is subjected to an inverse discrete wavelet transform using HH1, LH1 and HL1 subjected to weighting processing, and LL1. As a result, fine mucous membrane structure information is suppressed in a synthesized image signal (G signal).

On the other hand, an inverse discrete wavelet transform is performed on a B signal using HH2, LH2, HL2, HH3, LH3, HL3, HH4, LH4 and HL4 subjected to weighting processing, and HH1, LH1, HL1 and LL4. As a result, fine mucous membrane information is mainly reproduced in a synthesized image signal (B signal).

R, G and B signals thus processed are inputted to a γ circuit 75 shown in Fig. 10 to be subjected to a similar processing as described in the second embodiment.

According to the present embodiment, an image reproducing a mucous membrane structure with better image quality is obtained by using a separable two-dimensional filter. In addition, same working effects as in the second embodiment are obtained. Note that although, in the above description, a weighting factor of 1 is used when weighting is not performed, the weighting factor may be set to 1 or more to enhance contrast. That is, for a G signal, LL1 may be multiplied by a weighting factor of 1 or more to enhance contrast of image components composed of components in the low and middle frequency band, and for a B signal, HH1, LH1, and HL1 may be multiplied by a weighting factor of 1 or more to enhance contrast of fine mucous membrane information.

(Modification)

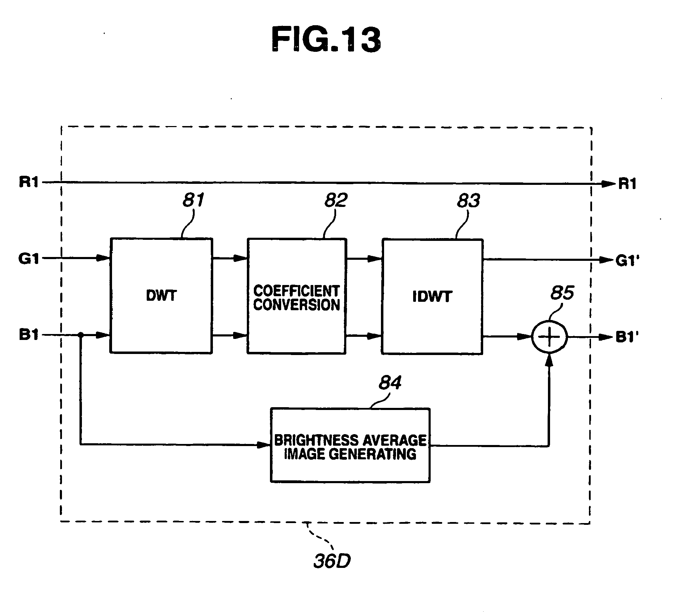

Fig. 13 shows a wavelet transform portion 36D according to a modification. The wavelet transform portion 36D is formed by providing a brightness average image generating circuit 84 which calculates a brightness average value of a B signal and an adder 85 which adds an output signal from the brightness average image generating circuit 84 and a B signal outputted from the IDWT 83 in the wavelet transform portion 36C in Fig. 11.

As in the configuration in Fig. 11, an R signal passes through the wavelet transform portion 36D without being processed and is outputted to the γ circuit 75, and G and B signals are sequentially inputted to the DWT 81, coefficient conversion circuit 82, and IDWT 83. The B signal is further inputted to the brightness average image generating circuit 84. An output signal from the brightness average image generating circuit 84 and an output signal from the IDWT 83 are added, and then a resultant signal is outputted to the γ circuit 75.

In the modification, the G and B signals are each decomposed into subbands of a same decomposition level (e.g., decomposition level 1) in the DWT 81.

In the coefficient conversion circuit 82, transform coefficients HH1, LH1 and HL1 are multiplied by weighting factors for a G signal to be reduced (e.g., uniformly multiplied by weighting factors of 0), and LL1 is multiplied by 1.

On the other hand, the coefficient conversion circuit 82 multiplies the coefficient LL1 in the B signal by a weighting factor of 0, and coefficients HH1, LH1, and HL1 by 1.

Coefficients having undergone weighting processing by the coefficient conversion circuit 82 are subjected to a two-dimensional inverse discrete wavelet transform in the IDWT 83. The B signal generates a synthetic image on the basis of LL1 weighted, and HH1, LH1 and HL1. The G signal also generates a synthetic image on the basis of LL1 weighted, and HH1, LH1 and HL1.

The brightness average image generating circuit 84 calculates a brightness average of a B signal and outputs an image signal in which pixels have a pixel value equal to the brightness average to all pixels. The image signal outputted from the brightness average image generating circuit 84 is inputted to the adder 85. Then, a B2 signal obtained by adding the image signal to the B signal outputted from the IDWT 83 is outputted from the wavelet transform portion 36D.

In the present modification, sharing of a decomposition level between G and B signals simplifies configuration, and provision of brightness average image generating means makes it possible to easily generate an image signal with better suppressed low frequency band components in a B signal. Moreover, the modification achieves the same effects as in the third embodiment.

Note that although the third embodiment and modification thereof have been described as being applied to the second embodiment, the third embodiment and modification thereof can also be applied to a frame sequential type.

In each of the above-described embodiments and the like, the living body observation apparatus may include only the video processor 4 or 4B having functions of signal processing means.

The above-described embodiments and the like are configured such that broadband illumination light generated by the light source device 3 or 3B is transmitted by the light guide 9, and the illumination light transmitted from the distal end surface of the light guide 9 through the illumination lens 23 is applied to a living mucous membrane or the like.

The present invention is not limited to the configuration. For example, the present invention may have a configuration in which a light-emitting element such as a light emitting diode (hereinafter abbreviated as an LED) is arranged at the distal end portion 22 of the electronic endoscope 2 or 2B to form illumination means, and a subject such as a living mucous membrane is illuminated by the light emitting element directly or through the illumination lens 23.

Note that an embodiment or the like which is configured by partially combining the above-described embodiments and the like also belongs to the present invention.

The present application is based on and claims priority from Japanese Patent Application No. 2006-058711 filed to Japan on March 3, 2006.

**Claims**

1. A living body observation apparatus (1, 1B) comprising signal processing means (4, 4B) capable of performing signal processing on an output signal from an image pickup device (25) which picks up an image under broadband illumination light to be applied to a living body and outputting a generated image signal to a display device side (5), and separation means (36, 36B, 36C, 36D) provided in the signal processing means (4, 4B), the separation means separating and extracting from the output signal a spatial frequency component corresponding to a structure of the living body,
   **characterized in that** the separation means (36, 36B, 36C, 36D) has a frequency characteristic which increases amplitudes in low and middle frequency bands and suppresses an amplitude in a high frequency band of a green signal outputted as the output signal.

2. The living body observation apparatus (1, 1B) according to claim 1, wherein the separation means (36, 36B, 36C, 36D) separates the output signal into a signal representing a fine mucous membrane structure and a coarse mucous membrane structure in the living body.

3. The living body observation apparatus (1, 1B) according to claim 1 or 2, wherein the signal processing means (4, 4B) comprises color adjustment means (38) for adjusting a tone of a separation means output signal outputted from the separation means (36, 36B, 36C, 36D).

4. The living body observation apparatus (1, 1B) according to any one of claims 1 to 3, wherein the signal processing means (4, 4B) comprises contrast conversion processing means (41) for subjecting the separation means output signal outputted from the separation means (36, 36B, 36C, 36D) to contrast conversion processing.

5. The living body observation apparatus (1, 1B) according to any one of claims 1 to 4, wherein the separation means (36, 36B, 36C, 36D) is configured using filters (52, 53) having different frequency pass characteristics corresponding to structures of the living body.

6. The living body observation apparatus (1, 1B) according to any one of claims 1 to 4, wherein the separation means (36, 36B, 36C, 36D) comprises wavelet transform meanss (36C, 36D).

7. The living body observation apparatus (1, 1B) according to any one of claims 1 to 4, wherein the separation means (36, 36B, 36C, 36D) is configured by a band-pass filter (52) set to have a frequency characteristic which increases amplitudes in low and middle frequency bands and suppresses an amplitude in a high frequency band of a green signal outputted as the output signal.

8. The living body observation apparatus (1, 1B) according to claim 7, wherein the separation means (36, 36B, 36C, 36D) comprises a high-pass filter (53) set to have a frequency characteristic which increases an amplitude in a high frequency band of a blue signal outputted as the output signal.

**Patentansprüche**

1. Vorrichtung zur Beobachtung eines lebenden Körpers (1, 1B) mit einem Signalverarbeitungsmittel (4, 4B), das in der Lage ist, eine Signalverarbeitung an einem Ausgangssignal eines Bildaufnahmegeräts (25) auszuführen, welches ein Bild unter Breitband-Beleuchtungslicht, das an einem lebenden Körper anzuwenden ist, aufnimmt, und ein erzeugtes Bildsignal an eine Anzeigegerät-Seite (5) auszugeben, und
einem in dem Signalverarbeitungsmittel (4, 4B) vorgesehenen Trennmittel (36, 36B, 36C, 36D), wobei das Trennmittel aus dem Ausgangssignal eine räumliche Frequenzkomponente aussondert und extrahiert, welche einer Struktur des lebenden Körpers entspricht,
**dadurch gekennzeichnet, dass** das Trennmittel (36, 36B, 36C, 36D) eine Frequenzcharakteristik aufweist, bei der Amplituden in Nieder- und Mittelfrequenzbändern vergrößert werden und eine Amplitude in einem Hochfrequenzband eines als das Ausgangssignal ausgegebenen grünen Signals gedämpft wird.

2. Vorrichtung zur Beobachtung eines lebenden Körpers (1, 1B) nach Anspruch 1, wobei das Trennmittel (36, 36B, 36C, 36D) das Ausgangssignal in ein Signal aufteilt, das eine feine Schleimhautstruktur und eine grobe Schleimhautstruktur in dem lebenden Körper darstellt.

3. Vorrichtung zur Beobachtung eines lebenden Körpers (1, 1B) nach Anspruch 1 oder 2, wobei das Signalverarbeitungsmittel (4, 4B) Farbanpassungsmittel (38) zum Anpassen eines Farbtons eines von dem Trennmittel (36, 36B, 36C, 36D) ausgegebenen Trennmittel-Ausgabesignals aufweist.

4. Vorrichtung zur Beobachtung eines lebenden Körpers (1, 1B) nach einem der Ansprüche 1 bis 3, wobei das Signalverarbeitungsmittel (4, 4B) Kontrastumwandlungs-Verarbeitungsmittel (41) aufweist, um ein von dem Trennmittel (36, 36B, 36C, 36D) ausgegebenes Trennmittel-Ausgabesignal einer Kontrastumwandlungsverarbeitung zu unterziehen.

5. Vorrichtung zur Beobachtung eines lebenden Körpers (1, 1B) nach einem der Ansprüche 1 bis 4, wobei das Trennmittel (36, 36B, 36C, 36D) unter Verwendung von Filtern (52, 53), die verschiedene, Strukturen des lebenden Körpers entsprechende Frequenzgang-Eigenschaften aufweisen, konfiguriert ist.

6. Vorrichtung zur Beobachtung eines lebenden Körpers (1, 1B) nach einem der Ansprüche 1 bis 4, wobei das Trennmittel (36, 36B, 36C, 36D) Wavelet-Transformationsmittel (36C, 36D) umfasst.

7. Vorrichtung zur Beobachtung eines lebenden Körpers (1, 1B) nach einem der Ansprüche 1 bis 4, wobei das Trennmittel (36, 36B, 36C, 36D) durch ein Bandpassfilter (52) konfiguriert ist, das so eingestellt ist, dass es eine Frequenzcharakteristik aufweist, bei der Amplituden in Nieder- und Mittelfrequenzbändern vergrößert werden und eine Amplitude in einem Hochfrequenzband eines als das Ausgabesignal ausgegebenen grünen Signals gedämpft wird.

8. Vorrichtung zur Beobachtung eines lebenden Körpers (1, 1B) nach Anspruch 7, wobei das Trennmittel (36, 36B, 36C, 36D) einen Hochpassfilter (53) aufweist, der so eingestellt ist, dass es eine Frequenzcharakteristik aufweist, bei der eine Amplitude in einem Hochfrequenzband eines als das Ausgabesignal ausgegebenen blauen Signals vergrößert wird.

## Revendications

1. Appareil d'observation d'un corps vivant (1, 1B) comprenant un moyen de traitement de signaux (4, 4B) apte à exécuter un traitement de signal sur un signal de sortie d'un dispositif de capture d'images (25) qui capture une image sous une lumière d'éclairage en bande large destinée à être appliquée à un corps vivant et à délivrer en sortie un signal d'image généré vers un côté de dispositif d'affichage (5), et
un moyen de séparation (36, 36B, 36C, 36D) prévu dans le moyen de traitement de signaux (4, 4B), le moyen de séparation séparant et extrayant du signal de sortie une composante de fréquence spatiale correspondant à une structure du corps vivant,
**caractérisé en ce que** le moyen de séparation (36, 36B, 36C, 36D) a une caractéristique de fréquence qui augmente des amplitudes dans des bandes de fréquences basses et moyennes et supprime une amplitude dans une bande de hautes fréquences d'un signal vert délivré en sortie comme le signal de sortie.

2. Appareil d'observation d'un corps vivant (1, 1B) selon la revendication 1, dans lequel le moyen de séparation (36, 36B, 36C, 36D) sépare le signal de sortie en un signal représentant une structure de muqueuse fine et une structure de muqueuse grossière dans le corps vivant.

3. Appareil d'observation d'un corps vivant (1, 1B) selon la revendication 1 ou 2, dans lequel le moyen de traitement de signaux (4, 4B) comprend un moyen d'ajustement de couleurs (38) pour ajuster une tonalité d'un signal de sortie de moyen de séparation délivré en sortie du moyen de séparation (36, 36B, 36C, 36D).

4. Appareil d'observation d'un corps vivant (1, 1B) selon l'une quelconque des revendications 1 à 3, dans lequel le moyen de traitement de signaux (4, 4B) comprend un moyen de traitement de conversion de contraste (41) pour soumettre le signal de sortie de moyen de séparation délivré en sortie du moyen de séparation (36, 36B, 36C, 36D) à un traitement de conversion de contraste.

5. Appareil d'observation d'un corps vivant (1, 1B) selon l'une quelconque des revendications 1 à 4, dans lequel le moyen de séparation (36, 36B, 36C, 36D) est configuré en utilisant des filtres (52, 53) ayant des caractéristiques passe-fréquence différentes correspondant à des structures du corps vivant.

6. Appareil d'observation d'un corps vivant (1, 1B) selon l'une quelconque des revendications 1 à 4, dans lequel le moyen de séparation (36, 36B, 36C, 36D) comprend un moyen de transformée en ondelettes (36C, 36D).

7. Appareil d'observation d'un corps vivant (1, 1B) selon l'une quelconque des revendications 1 à 4, dans lequel le moyen de séparation (36, 36B, 36C, 36D) est configuré par un filtre passe-bande (52) défini de façon à avoir une caractéristique de fréquence qui augmente des amplitudes dans des bandes de fréquences basses et moyennes et supprime une amplitude dans une bande de hautes fréquences d'un signal vert délivré en sortie comme le signal de sortie.

8. Appareil d'observation d'un corps vivant (1, 1B) selon la revendications 7, dans lequel le moyen de séparation (36, 36B, 36C, 36D) comprend un filtre passe-haut (53) défini de façon à avoir une caractéristique de fréquence qui augmente une amplitude dans une bande de hautes fréquences d'un signal bleu délivré en sortie comme le signal de sortie.

FIG.1

# FIG.2

WHITE LIGHT
BEAM

*14R*

*14B*

*14G*

*14*

# FIG.3

*14B*          *14G*          *14R*

B              G              R

TRANSMITTED LIGHT AMOUNT

380                                    700

WAVELENGTH
(nm)

# FIG.4

*51*

R

*52*
G          BPF

*53*
B          HPF

FROM TG 49          *36*

*37a*
R          R MEMORY          R'

*37b*
G MEMORY          BPF (G)

*37c*
B MEMORY          HPF (B)

*37*

COLOR CONVERSION CIRCUIT          R'

G'

B'

*38*

FRAME SEQUENTIAL CIRCUIT

*39*

16

# FIG.5

# FIG.6

# FIG.7

# FIG.8

# FIG.9

# FIG.10

EP 1 992 270 B1

# FIG.11

# FIG.12

# FIG.13

36D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002095635 A
- JP 2003093336 A
- EP 1576920 A1
- JP 4183430 A
- JP 2005293214 A
- US 2003139650 A1
- JP 2006058711 A